# EUROPEAN PATENT SPECIFICATION

(11) **EP 2 155 323 B1**
(45) Date of publication and mention of the grant of the patent: **08.05.2013**
(21) Application number: 08773512.2
(22) Date of filing: 19.06.2008
(51) Int. Cl.: A61N 1/20

(54) **PROCESS FOR REDUCING NEUROMUSCULAR FATIGUE CAUSED BY EXERCISE**
VERFAHREN ZUR REDUZIERUNG VON DURCH TRAINING VERURSACHTER NEUROMUSKULÄRER ERMÜDUNG
PROCEDE POUR LIMITER LA FATIGUE NEUROMUSCULAIRE PROVOQUEE PAR UN EXERCICE

(30) Priority: 21.06.2007 US 766401
(43) Date of publication of application: 24.02.2010
(73) Proprietor: Fondazione IRCCS "CA' GRANDA - OSPEDALE MAGGIORE POLICLINICO", 20122 Milan (IT)
(72) Inventor: COGIAMANIAN, Filippo, Maria, I-20015 Parabiago (Milano) (IT); PRIORI, Alberto, I-46030 Virgilio (Mantova) (IT); MARCEGLIA, Sara, Renata, Francesca, I-20122 Milano (IT)
(74) Representative: Biggi, Cristina
(86) International application number: PCT/EP2008/004924
(87) International publication number: WO 2008/155114

(56) References cited:
- US-A- 4 323 073
- US-A- 5 522 864
- US-A- 6 064 911

## Description

The present invention relates to a process for reducing neuromuscular fatigue caused by exercise, in particular for reducing fatigue in a living being such as a person about to participate in exercise or in a person having completed exercise.

Neuromuscular fatigue is the exercise dependent decrease in muscle ability to generate force. Therefore, in the context of the present invention, the term "fatigue" has to be intended as a condition, induced by the performance of exercises, which is a transitory physiological condition caused by a natural circumstance.

Simple training is generally known as retarding the perception of fatigue.

There also exist natural or synthetic drugs that reduce the fatigue caused by exercise, nevertheless many of such drugs involve addiction and cause adverse side effects.

The aim of the present invention is therefore to provide an alternative process for reducing neuromuscular fatigue caused by exercise.

This aim is achieved by the present invention, which involves manipulating brain excitability by anodal trans-cranial direct current stimulation, as disclosed in one or more of the appended claims.

In general the following method will be appliable to living being such as for example animals, i.e. a horse before a competition or any other animals before the execution of a task.

Hereinafter we will refer to a human being without anyway limiting the application to persons only.

An anodal trans-cranial direct current stimulation is a process of electric stimulation of a cortical region of a person's scalp (or animal, or living being) with an anodal direct current of low intensity, namely of low milliAmpere.

In particular, the process comprises applying an anodal trans-cranial direct current stimulation over the motor cortical areas of a person.

It has been found that the anodal trans-cranial direct current stimulation, described in detail below, improves muscle endurance, helping to start increased muscle endurance and decreasing muscle fatigue in normal, namely sports medicine, conditions.

The anodal trans-cranial direct current stimulation comprises applying an anodal electrode on the scalp of a person over the motor cortical areas.

Advantageously the anodal electrode is to be centred on the motor cortical areas of the scalp possibly to be active on both the right and the left motor cortical areas themselves.

It has been verified that good results may be also achieved placing the electrode specifically on the motor cortex.

In particular, in the test phase the anodal electrode was applied on the right motor cortex and in detail 4 centimetres laterally to the vertex.

A cathodal electrode is applied on a portion of the body of the same person; generally such an electrode is placed on a portion other than the scalp.

Preferably, the cathodal electrode is applied above the shoulder and, during the experimental tests, precisely on the right shoulder.

A direct current is then flowed from the cathodal electrode to the anodal electrode for a period between 5 and 60 minutes (in relation to the intensity used). Anyway a current flowing for a period between 5 to 30 minutes is generally sufficient.

The direct current intensity is chosen between 0.5 to 5 mA (in relation to the duration used), in order to polarise the brain, but a preferred upper limit is 3 mA.

The intensity and the standing of the direct current depend on various parameters, such as the physical features of the person, the position of the muscle on which the process has to be implemented and other.

In the preferred embodiment of the present invention, the intensity of the direct current is between 1 to 2 mA, preferably of 1,5 mA, for a period between 8 to 12 minutes, preferably of 10 minutes.

The direct current can be delivered by any suitable electrical device, for example by an electrical stimulator through a constant current unit and an isolation unit connected to said electrodes.

It is important that the direct current is an anodal current, since cathodal current does not achieve appreciable effects on the reduction of neuromuscular fatigue.

The electrodes may have any desirable shape even if a round shape gives better results with respect to squared or irregular shapes.

Of course a conductive material, such as a conductive gel or a saline composition, has to be used in combination with the electrodes.

The used electrodes have pieces of saline-soaked synthetic sponge in order to avoid possible harmful effects of high current density.

The preferred dimension for each electrode is a thickness of 0,3 centimetres and an area of 35 square centimetres even if other dimensions may be suitable as well.

In this regard, the direct current flowing from the cathodal electrode to the anodal electrode has a charge density that is between 0,01 to 0,5 Coulomb per square centimetre.

The range upper limit will be generally maintained under 0,2 Coulomb per square centimetre and more preferably the charge density is between 0,01 to 0,05 Coulomb per square centimetre; in the implemented method a charge density of 0,026 Coulomb per square centimetre was used.

Hereinafter the implementation of the process according to the present invention as performed on a group of 24 people is reported, by evaluating the fatigue reduction of left elbow flexors caused by exercise.

Said group consisted of 24 healthy right handed volunteers, in particular 14 women and 10 men having a mean age 24.3 years. None of the subjects engaged in competitive sport activities specifically involving elbow flexor muscles. The experimental procedures were in accordance with the declaration of Helsinki. Subjects sat in a chair with the left elbow on a padded support, the elbow joint at a right angle, and the wrist connected to a load cell. The motor task required the subject to exert an upward directed force with the wrist activating the elbow flexor muscles.

To determine the maximal voluntary contraction force, subjects were instructed to increase the force from zero to a maximum level and to hold it for 3 seconds maximal voluntary contraction. Subjects performed nine maximal voluntary contraction trials subdivided in three sessions and rested for 60 sec between each trial. The mean of the best three performances was taken as the maximal voluntary contraction force and used as the reference to calculate the target force for the fatigue contraction.

Muscle endurance was assessed as the time subjects could sustain an isometric contraction with the elbow flexor muscles at 35% of the maximal voluntary contraction force as determined previously. During the fatigue task, each subject received force feedback on a computer monitor showing a 35% maximal voluntary contraction target. The subject was encouraged to keep the force at this level.

The contraction was terminated when the subject deviated from the target force for more than 3 seconds despite strong verbal encouragement.

Trans-cranial magnetic stimulation was delivered by a stimulator through a flat coil centred over the vertex with currents flowing clockwise (viewed from above). Motor evoked potentials were recorded by standard non-polarizable Ag-AgCl surface electrodes placed over the belly of the biceps brachii muscle and on the skin overlying the biceps' tendon of the left arm. The motor threshold was defined as the lowest intensity able to produce motor evoked potentials of ≥ 50 µV in 5 out of 10 consecutive trials of stimulation during a slight contraction of biceps. Stimulation intensity was 120% of the baseline motor evoked potential threshold. A total of 16 motor evoked potentials were recorded in response to 16 stimuli delivered at 0.15 Hz. The peak-to-peak amplitude was measured before and immediately after trans-cranial direct current stimulation.

The trans-cranial direct current stimulation had an intensity of 1,5 mA and a duration of 10 minutes. It was delivered by an electrical stimulator through a constant current unit and an isolation unit connected to a pair of electrodes. One electrode was placed on the scalp over the right motor cortex, in particular 4 cm laterally to the vertex and the other electrode was placed above the right shoulder. Stimulating electrodes were pieces of saline-soaked synthetic sponge. To guarantee safety, the density charge was of 0.026 C/cm². These criteria are far below the threshold for tissue damage. The wide electrode surface avoided the possible harmful effects of high current density. Apart from occasional, transient and short lasting tingling and burning sensations below the electrodes, direct current stimulation strength remained below the conscious cutaneous sensory threshold throughout the experimental session. Direct current stimulation polarity (cathodal or anodal) refers to the electrode over the right motor area.

The effect of anodal and cathodal trans-cranial direct current stimulation on maximal voluntary contraction and endurance time was evaluated. Three levels, namely anodal trans-cranial direct current stimulation, cathodal trans-cranial direct current stimulation and independent measures without stimulation were performed.

No difference was noted between normalized endurance time values at 1 hour after cathodal trans-cranial direct current stimulation and measures without stimulation. Anodal trans-cranial direct current stimulation induced a significant decrease in endurance time at 1 hour when compared to measures without stimulation and cathodal trans-cranial direct current stimulation.

In particular, at 1 hour after the baseline fatigue task, the expected shortening of the endurance time, owing to residual tiredness, is reduced by about 15% after anodal trans-cranial direct current stimulation compared with cathodal trans-cranial direct current stimulation and measures without stimulation.

The example above disclosed shows that anodal trans-cranial direct current stimulation applied over the right motor cortical areas prolongs the endurance time for contralateral elbow flexors in a submaximal isometric task.

Analogous effects are achieved on all the other muscles of the people subjected to the anodal trans-cranial direct current stimulation, in particular when the direct current has been applied over the right motor cortical areas.

Moreover, anodal trans-cranial direct current stimulation improves muscle performance and decreasing muscle fatigue both in normal (conditions which do not require any therapeutic treatment) and pathological conditions, for example in stroke and primary or secondary chronic fatigue syndrome rehabilitation.

Methods of treatment or therapy or rehabilitation are not claimed, however.

## Claims

1. A process for reducing the perception of neuromuscular fatigue caused by an exercise in a healthy person, comprising the steps of:
(i) having a person participating in an exercise or a person having completed an exercise said exercise causing a neuromuscular fatigue to said person;
(ii) placing at least one anodal electrode on the scalp of the fatigued person of step (i) over a motor cortex area, and at least one cathodal electrode on a portion of the body other than the scalp of the same person; and
(iii) applying an anodal trans-cranial direct current stimulation between the electrodes of step (ii).

2. The process according to claim 1, wherein the cathodal electrode is applied on the right shoulder.

3. The process according to claim 1 or 2, wherein the anodal electrode is applied on the right motor cortex, preferably four centimetres laterally to the vertex.

4. The process according to any one of claims 1-3 comprising making a direct current flow from the cathodal electrode to the anodal electrode, said direct current having an intensity between 0.5 to 5 mA for a period between 5 to 30 minutes.

5. The process according to claim 4, wherein the intensity of the direct current is between 1 to 2 mA for a period between 8 to 12 minutes.

6. The process according to claim 5, wherein the intensity of the direct current is of 1,5 mA for a period of 10 minutes.

7. The process according to any one of claims 1-6, comprising flowing said direct current having a charge density comprised between 0,010 to 0,5 C/cm².

8. The process according to claim 7, wherein said charge density is comprised between 0,010 to 0,05 C/cm².

9. The process according to claim 8, wherein said charge density is 0,026 C/cm².

10. The process according to any one of claims 1-9, comprising providing said electrodes having a round shape.

11. The process according to any one of claims 1-10, comprising providing said electrodes having pieces of saline-soaked synthetic sponge.

## Patentansprüche

1. Verfahren zur Reduzierung der Wahrnehmung von durch Training verursachter neuromuskulärer Ermüdung bei einer gesunden Person, umfassend folgende Schritte:
(i) eine Person zur Verfügung haben, die an einem Training teilnimmt, oder eine Person, die ein Training abgeschlossen hat, wobei dieses Training bei der betreffenden Person eine neuromuskuläre Ermüdung verursacht;
(ii) Anbringen von mindestens einer Anoden-Elektrode an der Kopfhaut der ermüdeten Person aus Schritt (i) über einem Motorcortexbereich und von mindestens einer Kathoden-Elektrode an einem Abschnitt des Körpers, der nicht die Kopfhaut ist, derselben Person, und
(iii) Aufbringen einer anodalen transkraniellen Gleichstromstimulation zwischen den Elektroden aus Schritt (ii).

2. Verfahren nach Anspruch 1, wobei die Kathoden-Elektrode an der rechten Schulter angebracht wird.

3. Verfahren nach Anspruch 1 und 2, wobei die Anoden-Elektrode am rechten Motorcortex angebracht wird, vorzugsweise vier Zentimeter seitlich des Scheitels.

4. Verfahren nach einem der Ansprüche 1 bis 3, umfassend, dass dafür gesorgt wird, dass ein Gleichstrom von der Kathoden-Elektrode zur Anoden-Elektrode fließt und dieser Gleichstrom eine Stärke von 0,5 bis 5 mA für einen Zeitraum von 5 bis 30 Minuten aufweist.

5. Verfahren nach Anspruch 4, wobei die Stärke des Gleichstroms zwischen 1 und 2 mA für einen Zeitraum von 8 bis 12 Minuten beträgt.

6. Verfahren nach Anspruch 5, wobei die Stärke des Gleichstroms 1,5 mA für einen Zeitraum von 10 Minuten beträgt.

7. Verfahren nach einem der Ansprüche 1 bis 6, umfassend, dass der fließende Gleichstrom eine Ladungsdichte zwischen 0,010 und 0,5 C/cm² aufweist.

8. Verfahren nach Anspruch 7, wobei die Ladungsdichte zwischen 0,010 und 0,05 C/cm² beträgt.

9. Verfahren nach Anspruch 8, wobei die Ladungsdichte 0,026 C/cm² beträgt.

10. Verfahren nach einem der Ansprüche 1 bis 9, umfassend die Bereitstellung der Elektroden, die eine runde Form aufweisen.

11. Verfahren nach einem der Ansprüche 1 bis 10, umfassend die Bereitstellung der Elektroden, die Teile eines mit Kochsalzlösung getränkten synthetischen Tupfers aufweisen.

## Revendications

1. Procédé pour limiter la perception de la fatigue neuromusculaire provoquée par un exercice chez une personne en bonne santé comprenant les étapes de :
(i) disposer d'une personne participant à un exercice ou une personne ayant terminé un exercice ; ledit exercice provoquant une fatigue neuromusculaire à ladite personne ;
(ii) placer au moins une électrode anodique sur le cuir chevelu de la personne fatiguée de l'étape (i) sur une région motrice du cortex, et au moins une électrode cathodique sur une partie du corps autre que celle du cuir chevelu de la même personne ; et
(iii) appliquer une excitation par courant continu transcrânienne anodique entre les électrodes de l'étape (ii).

2. Procédé selon la revendication 1, dans lequel l'électrode cathodique est appliquée sur l'épaule droite.

3. Procédé selon les revendications 1 ou 2, dans lequel l'électrode anodique est appliquée sur le cortex moteur droit, de préférence quatre centimètres latéralement par rapport au vertex.

4. Procédé selon les revendications de 1 à 3 comprenant la réalisation d'un passage de courant continu de l'électrode cathodique à l'électrode anodique, ledit courant continu ayant une intensité comprise entre 0,5 et 5 mA pendant une période comprise entre 5 et 30 minutes.

5. Procédé selon la revendication 4, dans lequel l'intensité du courant continu est comprise entre 1 et 2 mA pendant une période comprise entre 8 et 12 minutes.

6. Procédé selon la revendication 5, dans lequel l'intensité du courant continu est de 1,5 mA pendant une période de 10 minutes.

7. Procédé selon les revendications de 1 à 6, comprenant l'écoulement dudit courant continu ayant une densité de charge comprise entre 0,010 et 0,5 C/cm².

8. Procédé selon la revendication 7, dans lequel ladite densité de charge est comprise entre 0,010 et 0,05 C/cm².

9. Procédé selon la revendication 8, dans lequel ladite densité de charge est de 0,026 C/cm².

10. Procédé selon l'une quelconque des revendications de 1 à 9, comprenant la fourniture desdites électrodes ayant une forme arrondie.

11. Procédé selon l'une quelconque des revendications de 1 à 10, comprenant la fourniture desdites électrodes ayant des morceaux d'éponge synthétiques imbibés de solution saline.
